# FASCICULE DE BREVET EUROPEEN

(11) **EP 1 708 760 B1**
(45) Date de publication et mention de la délivrance du brevet: **27.03.2013**
(21) Numéro de dépôt: 05717407.0
(22) Date de dépôt: 13.01.2005
(51) Int. Cl.: A61L 2/08

(54) **INSTALLATION DE STERILISATION D'ARTICLES PAR BOMBARDEMENT ELECTRONIQUE**
VORRICHTUNG ZUM STERILISIEREN VON GEGENSTÄNDEN MIT ELEKTRONENBOMBARDIERUNG
DEVICE FOR STERILISING ARTICLES BY ELECTRON BOMBARDMENT

(30) Priorité: 20.01.2004 FR 0400473
(43) Date de publication de la demande: 11.10.2006
(73) Titulaire: SERAC GROUP, 72400 La Ferté-Bernard (FR)
(72) Inventeur: DUMARGUE, Guy, F-72400 CHERRE (FR); GRUSON, Bertrand, F-50290 BREVILLE SUR MER (FR); RAYNAUD, Delphine, F-72400 LA FERTE BERNARD (FR)
(74) Mandataire: Fruchard, Guy
(86) Numéro de dépôt international: PCT/FR2005/000072
(87) Numéro de publication internationale: WO 2005/079870

(56) Documents cités:
- WO-A-98/42385
- FR-A- 2 838 403
- US-A- 4 944 132
- US-A1- 2002 149 321
- US-B1- 6 191 424

## Description

La présente invention concerne une installation de stérilisation d'articles, plus particulièrement bien que non exclusivement des bouteilles.

### ARRIERE PLAN DE L'INVENTION

On connaît des installations de stérilisation d'articles, notamment des barquettes de produits alimentaires, l'installation comprenant un enceinte traversée par un transporteur sur lequel sont disposés les articles, et un dispositif de bombardement électronique disposé au-dessus du transporteur pour traiter les articles par un rayonnement vertical. Ces installations sont satisfaisantes pour des articles ayant une faible hauteur, ce qui permet d'assurer une stérilisation de l'article en utilisant un rayonnement électronique de faible énergie.

En revanche, lorsque les articles ont une hauteur et/ou une épaisseur importantes, par exemple dans le cas de bouteilles, il est nécessaire d'augmenter de façon très importante l'énergie du bombardement électronique afin que le rayonnement électronique atteigne le fond de la bouteille après avoir traversé la paroi, en ayant encore une énergie suffisante pour assurer une stérilisation du fond du récipient. Dans le cas de bouteilles, l'énergie du bombardement électronique doit être d'autant plus importante que le col de la bouteille absorbe une partie de l'énergie du rayonnement électronique avant que celui-ci atteigne le fond de la bouteille. Un bombardement électronique de puissance élevé pose alors un problème de dégradation des parties de l'article les plus proches du dispositif de bombardement, en particulier le col dans le cas d'une bouteille.

On connaît en outre du document US 4 944 132 une installation de stérilisation comportant deux organes de bombardement électronique disposés selon des orientations différentes par rapport aux articles à stériliser mais émettant tous les deux un rayonnement horizontal sur des récipients s'étendant verticalement. Le fond d'une bouteille ne serait donc pas convenablement stérilisé avec une installation conforme à ce document.

En outre, un bombardement électronique de forte puissance risque de se propager à l'extérieur de l'enceinte et constitue donc un danger pour les opérateurs circulant autour de l'installation.

### OBJET DE L'INVENTION

Un but principal de l'invention est de proposer une installation de stérilisation d'articles permettant d'assurer une stérilisation intérieure et extérieure suffisante des articles par un bombardement électronique tout en minimisant les dégradations de l'article par le bombardement électronique.

D'autres aspects de l'invention visent à minimiser la propagation du rayonnement électronique à l'extérieur de l'enceinte et minimiser la pénétration de pollution à l'intérieur de l'enceinte.

### BREVE DESCRIPTION DE L'INVENTION

En vue de la réalisation du but principal de l'invention, on propose selon l'invention, une installation de stérilisation d'articles comprenant une enceinte comprenant une ouverture d'entrée et une ouverture de sortie, un dispositif support pour déplacer les articles dans l'enceinte avec un axe longitudinal des articles s'étendant verticalement, et au moins deux organes de bombardement électronique disposés selon des orientations différentes par rapport aux articles à stériliser pour traiter les articles pendant leur déplacement dans l'enceinte, caractérisée en ce que les organes de bombardement électronique sont inclinés par rapport a la verticale pour émettre un rayonnement électronique incliné par rapport à la verticale.

Ainsi, par un positionnement approprié des organes de bombardement électronique en fonction de la forme de l'article à stériliser, il est possible de s'assurer que l'organe de bombardement électronique est en regard d'une partie de l'article de faible épaisseur, de sorte qu'il est possible d'assurer une stérilisation de chaque partie de l'article avec un bombardement électronique de faible énergie. On obtient alors une stérilisation totale de l'article, y compris le fond d'un récipient sans dégradation de l'article.

Selon un autre aspect de l'invention, le dispositif support comporte au moins deux organes supports disposés pour supporter les récipients selon des positions variées correspondant aux organes de bombardement électronique, de préférence, les organes supports sont des plates-formes rotatives disposées pour faire suivre aux articles un parcours en S. On s'assure ainsi que les organes supports ne forment pas des obstacles à la propagation du rayonnement lors du passage de l'article devant l'organe de bombardement électronique correspondant.

Selon encore un autre aspect avantageux de l'invention, l'enceinte comporte des parois latérales présentant un profil curviligne ou en ligne brisée. On s'assure ainsi que le rayonnement électronique est soumis à un nombre important de réflexions sur les parois de l'enceinte avant d'atteindre l'ouverture d'entrée ou l'ouverture de sortie, de sorte que le rayonnement sortant de l'enceinte a une énergie résiduelle suffisamment faible pour ne pas constituer un danger pour les opérateurs circulant autour de l'installation.

Selon encore d'autres aspects avantageux de l'invention, l'enceinte comporte des éléments de parois disposés pour minimiser la circulation d'air dans l'enceinte et l'installation comporte un dispositif d'injection de gaz stérile à l'intérieur de l'enceinte, de préférence disposé à l'aplomb des articles. On minimise ainsi tout à la fois la pénétration d'air pollué depuis l'extérieur de l'enceinte et l'émission du rayonnement électronique vers l'extérieur de l'enceinte.

### BREVE DESCRIPTION DES DESSINS

D'autres caractéristiques et avantages de l'invention apparaîtront à la lecture de la description qui suit d'un mode de réalisation préféré non limitatif de l'invention en relation avec les figures ci-jointes parmi lesquelles :
- la figure 1 est une vue de dessus schématique de l'installation selon l'invention,
- la figure 2 est une vue en coupe schématique selon la ligne II-II de la figure 1 de l'installation en position de fonctionnement,
- la figure 3 est une vue en coupe analogue à celle de la figure 2 de l'installation dans une position d'intervention.

### DESCRIPTION DETAILLEE DE L'INVENTION

L'invention est décrite ci-dessous en relation avec une installation de stérilisation de bouteilles 1.

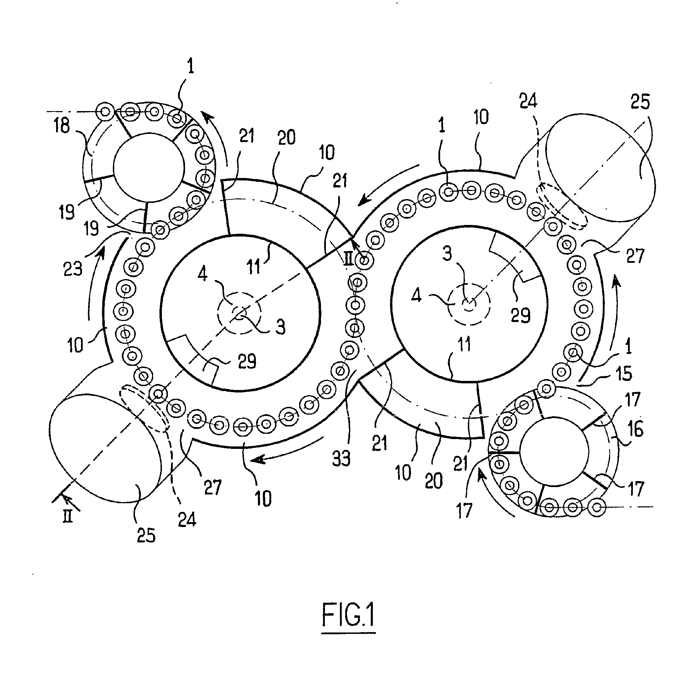

Dans le mode de réalisation illustré, l'installation comporte deux organes supports de bouteilles comprenant chacun de façon connue en soi une plate-forme rotative 2 (non représentée sur la figure 1), supportée par un axe 3 entraîné en rotation par un moteur 4 porté par un châssis 5. La plate-forme rotative 2 comporte de façon également connue en soi des pinces de préhension 6 permettant de saisir les bouteilles 1 sur une partie du pourtour du col des bouteilles en laissant le col dégagé vers l'extérieur de la plate-forme.

Les plates-formes rotatives 2 sont disposées adjacentes l'une à l'autre et sont entraînées en rotation dans des sens inverses. Les pinces de préhension 6 sont en outre commandées de façon connue en soi par un organe de commande non représenté permettant de transférer les bouteilles d'une plate-forme rotative à l'autre lors du passage au point de tangence des plates-formes rotatives de sorte que les bouteilles suivent une trajectoire en S comme illustré sur la figure 1.

Une enceinte 7 s'étend autour des organes supports en délimitant autour des récipients un volume permettant un déplacement des récipients dans l'enceinte lorsque ceux-ci sont portés par les pinces 6.

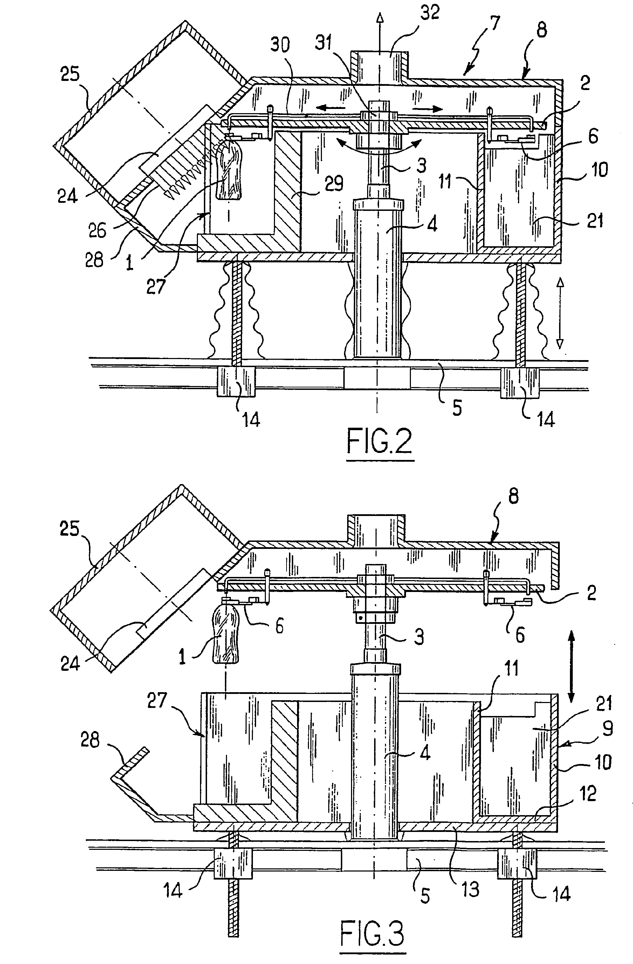

Comme illustré, l'enceinte 7 comporte de préférence un couvercle 8 fixé au châssis de l'installation par des moyens non représentés, et une cuve 9 comportant en relation avec chaque plate-forme rotative 2 une paroi latérale externe 10 cylindrique et une paroi latérale interne 11 cylindrique reliées à un fond 12. De part et d'autre du point de tangence des plates-formes rotatives 1, les parois latérales externes 10 sont interrompues et sont réunies l'une à l'autre de façon étanche pour délimiter une ouverture de liaison 33 entre les deux parties d'enceinte chacune associée à une plate-forme rotative 2. La cuve 9 est portée par une plaque 13 mobile verticalement, reliée au châssis 5 par des vérins à vis 14 permettant de déplacer la cuve entre une position ouverte illustrée sur la figure 3 dans laquelle la cuve 9 est en position basse permettant d'accéder aux pinces 6 et à la plate-forme 2, et une position de fonctionnement illustrée sur la figure 2 dans laquelle la paroi externe 10 de la cuve est en contact étanche aux radiations avec le couvercle 8, par exemple en utilisant un joint à chicane, et la paroi latérale interne 11 a un bord supérieur s'étendant sous la plate-forme 2 à proximité de celle-ci pour permettre une rotation sans frottement de la plate-forme 2 tout en réalisant un écran au rayonnement électronique qui va être décrit ci-après. L'enceinte est de préférence réalisée en acier avec un revêtement interne en plomb.

La paroi externe 10 de l'enceinte est percée d'une ouverture d'entrée 15 en regard de laquelle s'étend un dispositif rotatif 16 d'introduction des bouteilles, par exemple une étoile de transfert, comportant des cloisons radiales 17 espacées d'une distance suffisamment faible pour que deux cloisons radiales 17 soient en permanence voisines des bords de l'ouverture 15. Les cloisons radiales 17 minimisent ainsi les risques de pénétration de l'air externe pollué à l'intérieur de l'enceinte, et constituent en outre des obstacles à la sortie du rayonnement électronique réfléchi à l'intérieur de l'enceinte. Dans une zone opposée de l'enceinte, l'installation comporte un dispositif rotatif 18 d'extraction des bouteilles depuis l'enceinte. Le dispositif rotatif d'extraction 18 s'étend en regard d'une ouverture de sortie 23 réalisée dans la paroi latérale externe 10 de l'enceinte. De même que le dispositif rotatif 16, le dispositif rotatif 18 comporte des cloisons radiales 19 formant des obstacles à la pénétration de l'air extérieur à l'intérieur de l'enceinte. En outre, aux extrémités des zones mortes 20 dans lesquelles les pinces 6 ne portent pas de bouteille, la cuve comporte des cloisons radiales 21 découpées pour permettre la rotation de la plate-forme 2 et des pinces 6 associées tout en minimisant le risque de pénétration d'air extérieur pollué en passant par la zone morte 20 correspondante.

Dans deux zones radialement opposées par référence à la trajectoire des bouteilles 1, l'installation comporte deux organes de bombardement électronique 24 chacun disposé dans un boîtier 25 fixé au couvercle 8 pour émettre un rayonnement électronique 26 à 45° par rapport à l'axe longitudinal vertical des bouteilles 1. Le rayonnement électronique 26 pénètre dans l'enceinte 7 par une ouverture 27 dans la paroi latérale externe 10 de l'enceinte. De même que l'enceinte, le boîtier 25 est de préférence réalisé avec un revêtement interne en plomb. Afin d'assurer la continuité entre le boîtier 25 et la cuve de l'enceinte, celle-ci comporte un élément de carter 28 de forme appropriée solidaire de la cuve 9. En regard de l'organe de bombardement électronique 24, la paroi interne 11 de la cuve 9 est de préférence renforcée par une pièce en plomb 29 absorbant le rayonnement électronique direct afin d'éviter la propagation de celui-ci à travers la paroi latérale interne de l'enceinte.

Par ailleurs, une canne d'injection 30 est associée à chaque pince de préhension 6. Chaque canne d'injection 30 a une extrémité débouchant à l'aplomb d'une bouteille 1, et une extrémité opposée reliée à un joint tournant 31 permettant une alimentation de la canne d'injection 30 en gaz stérile sous pression à partir d'une source de gaz non représentée. Le gaz stérile est de préférence de l'azote qui permet de réduire la production d'ozone lors du bombardement électronique. Le gaz stérile sous pression réalise en outre à l'intérieur de l'enceinte 7 une surpression minimisant la pénétration de l'air extérieur. Pour éviter une surpression trop importante à l'intérieur de l'enceinte, le couvercle 8 est de préférence équipé d'un orifice d'extraction 32.

Bien entendu, l'invention n'est pas limitée au mode de réalisation illustré et est susceptible de variantes de réalisation sans sortir du cadre de l'invention tel que défini par les revendications.

En particulier, bien que l'installation de stérilisation ait été décrite pour la stérilisation de bouteilles elle peut être utilisée pour la stérilisation d'articles quelconques, ces articles étant supportés par un dispositif support approprié. Bien que l'installation selon l'invention ait été illustrée avec deux organes de bombardement électronique associés à deux organes supports rotatifs, le nombre d'organes de bombardement électronique sera adapté à la forme et à la dimension des articles pour assurer la stérilisation souhaitée tout en minimisant le nombre d'organes de bombardement électronique. On peut également associer plusieurs organes de bombardement électronique à un même organe support si la configuration de l'organe support et la forme des articles à stériliser le permet.

Bien que l'enceinte selon l'invention ait été décrite avec des parois latérales cylindriques, on pourra utiliser une enceinte comportant des parois latérales présentant un profil curviligne ou en ligne brisée de façon à assurer des réflexions successives du rayonnement électronique sur les parois de l'enceinte et affaiblir ainsi l'intensité du rayonnement avant que celui-ci atteigne l'orifice d'entrée ou l'orifice de sortie de l'enceinte.

Bien que l'invention ait été décrite en relation avec des dispositifs de bombardement électronique dont le rayonnement émis est orienté à 45°, la valeur de cette inclinaison dans la plage entre 0° et 90° par rapport à la direction verticale des axes longitudinaux des articles, sera adaptée à la forme des articles à stériliser, et à l'épaisseur de la paroi dans le cas des récipients.

## Revendications

1. Installation de stérilisation d'articles (1) comprenant une enceinte (7) comprenant une ouverture d'entrée (15) et une ouverture de sortie (23), un dispositif support (2) pour déplacer les articles (1) dans l'enceinte avec un axe longitudinal des articles s'étendant verticalement, et au moins deux organes de bombardement électronique (24) disposés selon des orientations différentes par rapport aux articles à stériliser pour traiter les articles (1) pendant leur déplacement dans l'enceinte, **caractérisée en ce que** les organes de bombardement électronique (24) sont inclinés par rapport la vertical pour émettre un rayonnement électronique (26) incliné par rapport à la verticale.

2. Installation selon la revendication 1, **caractérisée en ce que** le dispositif support comporte au moins deux organes supports (2) disposés pour supporter les récipients selon des positions variées correspondant aux organes de bombardement électronique (24).

3. Installation selon la revendication 2, **caractérisée en ce que** les organes supports sont des plates-formes rotatives (2) disposées pour faire suivre aux articles (1) un parcours en S.

4. Installation selon la revendication 1, **caractérisée en ce que** l'enceinte a des parois latérales (10, 11) présentant un profil curviligne ou en ligne brisée.

5. Installation selon la revendication 1, **caractérisée en ce que** le dispositif support comporte au moins une plate-forme rotative pour supporter les articles, et **en ce que** l'enceinte (7) comporte des parois latérales cylindriques (10, 11) entre lesquelles les articles (1) se déplacent.

6. Installation selon la revendication 5, **caractérisée en ce que** l'enceinte comporte une zone morte fermée par des cloisons radiales (21).

7. Installation selon la revendication 5, **caractérisée en ce qu'**elle comporte des dispositifs rotatifs (16, 18) d'introduction d'articles et d'extraction d'articles, ces dispositifs rotatifs comportant des cloisonnements radiaux (17, 19).

8. Installation selon la revendication 1, **caractérisée en ce que** l'enceinte comporte au moins deux parties (8, 9) mobiles l'une par rapport à l'autre pour permettre un accès aux articles.

9. Installation selon la revendication 1, **caractérisée en ce qu'**elle comporte un dispositif (30) d'injection de gaz stérile à l'intérieur de l'enceinte.

10. Installation selon la revendication 9, **caractérisée en ce que** le dispositif d'injection de gaz stérile comporte des cannes d'injection (30) ayant une extrémité s'étendant à l'aplomb des articles (1).

## Claims

1. An installation for sterilizing articles (1), the installation comprising an enclosure (7) having an inlet opening (15) and an outlet opening (23), a support device (2) for moving the articles (1) inside the enclosure with a vertically extending longitudinal axis of the articles, and at least two electron bombardment members (24) disposed at different orientations relative to the articles that are to be sterilized to process the articles (1) as they move through the enclosure, **characterized in that** the electron bombardment members (24) are inclined relative to a vertical line in order to limit an electron radiation (26) inclined relative to the vertical line.

2. An installation according to claim 1, **characterized in that** the support device comprises at least two support members (2) disposed to support the receptacles in various positions corresponding to respective electron bombardment members (24).

3. An installation according to claim 2, **characterized in that** the support members are rotary platforms (2) disposed to cause the articles (1) to follow an S-shaped path.

4. An installation according to claim 1, **characterized in that** the enclosure has side walls (10, 11) presenting a curvilinear or zigzag profile.

5. An installation according to claim 1, **characterized in that** the support device comprises at least one rotary platform for supporting the articles, and **in that** the enclosure (7) has cylindrical side walls (10, 11) between which the articles (1) move.

6. An installation according to claim 5, **characterized in that** the enclosure includes a dead zone closed by radial partitions (21).

7. An installation according to claim 5, **characterized in that** it includes rotary devices (16, 18) for introducing articles and for extracting articles, the rotary devices including radial partitions (17, 19).

8. An installation according to claim 1, **characterized in that** the enclosure comprises at least two portions (8, 9) that are movable relative to each other in order to provide access to the articles.

9. An installation according to claim 1, **characterized in that** it includes a device (30) for injecting sterile gas into the inside of the enclosure.

10. An installation according to claim 9, **characterized in that** the device for injecting sterile gas comprises injection pipes (30) each having one end extending vertically above the articles (1).

## Patentansprüche

1. Anordnung zum Sterilisieren von Gegenständen (1) umfassend einen Behälter (7), der eine Eingangsöffnung (15) und eine Ausgangsöffnung (23), eine Trägervorrichtung (2) zum Verlagern der Gegenstände (1) in dem Behälter, wobei sich eine Längsachse der Gegenstände vertikal erstreckt, und mindestens zwei Elektronenbeschussorgane (24) enthält, die bezüglich der zu sterilisierenden Gegenstände unterschiedlich ausgerichtet sind, um die Gegenstände (1) während ihrer Verlagerung in dem Behälter zu behandeln, **dadurch gekennzeichnet, dass** die Elektronenbeschussorgane (24) gegenüber der Vertikalen geneigt sind, um eine Elektronenstrahlung (26) auszusenden, die gegenüber der Vertikalen geneigt ist.

2. Anordnung nach Anspruch 1, **dadurch gekennzeichnet, dass** die Trägervorrichtung mindestens zwei Trägerorgane (2) umfasst, die so angeordnet sind, dass sie die Gefäße in unterschiedlichen Positionen, die den Elektronenbeschussorganen (24) entsprechen, halten.

3. Anordnung nach Anspruch 2, **dadurch gekennzeichnet, dass** die Trägerorgane rotierende Plattformen (2) sind, die so angeordnet sind, dass die Gegenstände (1) eine S-förmige Bahn durchlaufen.

4. Anordnung nach Anspruch 1, **dadurch gekennzeichnet, dass** der Behälter Seitenwände (10, 11) besitzt, die ein gekrümmtes Profil oder eine gebrochene Linie aufweisen.

5. Anordnung nach Anspruch 1, **dadurch gekennzeichnet, dass** die Trägervorrichtung mindestens eine rotierende Plattform zum Halten der Gegenstände umfasst und dass der Behälter (7) zylindrische Seitenwände (10, 11) aufweist, zwischen denen die Gegenstände (1) verlagert werden.

6. Anordnung nach Anspruch 5, **dadurch gekennzeichnet, dass** der Behälter eine Totzone umfasst, die von radialen Zwischenwänden (21) abgeschlossen ist.

7. Anordnung nach Anspruch 5, **dadurch gekennzeichnet, dass** sie rotierende Vorrichtungen (16, 18) zum Einführen der Gegenstände und zum Entfernen der Gegenstände umfasst, wobei diese rotierenden Vorrichtungen radiale Trennwände (17, 19) aufweisen.

8. Anordnung nach Anspruch 1, **dadurch gekennzeichnet, dass** der Behälter mindestens zwei zueinander bewegliche Abschnitte (8, 9) umfasst, um einen Zugang zu den Gegenständen zu ermöglichen.

9. Anordnung nach Anspruch 1, **dadurch gekennzeichnet, dass** sie eine Vorrichtung (30) zum Einblasen von sterilem Gas in das Innere des Behälters umfasst.

10. Anordnung nach Anspruch 9, **dadurch gekennzeichnet, dass** die Vorrichtung zum Einblasen von sterilem Gas Injektionsrohre (30) mit einem Ende umfasst, das sich senkrecht über den Gegenständen (1) erstreckt.
